# EUROPÄISCHE PATENTSCHRIFT

(11) **EP 2 624 770 B1**
(45) Veröffentlichungstag und Bekanntmachung des Hinweises auf die Patenterteilung: **03.12.2014**
(21) Anmeldenummer: 11740656.1
(22) Anmeldetag: 04.08.2011
(51) Int. Cl.: A61B 17/122

(54) **CHIRURGISCHER CLIP**
SURGICAL CLIP
CLAMP CHIRURGICAL

(30) Priorität: 04.10.2010 DE 102010037949
(43) Veröffentlichungstag der Anmeldung: 14.08.2013
(73) Patentinhaber: Aesculap AG, 78532 Tuttlingen (DE)
(72) Erfinder: WEISSHAUPT, Dieter, 78194 Immendingen (DE)
(74) Vertreter: Hoeger, Stellrecht & Partner Patentanwälte
(86) Internationale Anmeldenummer: PCT/EP2011/063488
(87) Internationale Veröffentlichungsnummer: WO 2012/045500

(56) Entgegenhaltungen:
- DE-A1- 19 907 354
- US-A- 4 943 298
- US-A- 5 207 692
- US-A- 5 944 729

## Beschreibung

Die Erfindung betrifft einen chirurgischen Clip, insbesondere zur Verwendung als Implantat, wobei hier die Verwendung als cerebraler Aneurysmen-Clip besondere Bedeutung hat.

Chirurgische Clips mit zwei Clipbranchen und einem federelastischen Element, das die beiden Clipbranchen an einem ihrer Enden miteinander verbindet, sind bekannt.

Die beiden Clipbranchen weisen jeweils ein erstes freies Ende auf, welche in Ruhestellung des Clips mit einer vorgegebenen Schließkraft durch das federelastische Element parallel aneinander anliegend gehalten sind, während ein zweites, dem jeweiligen freien Ende entgegen gesetztes Ende an dem federelastischen Element gehalten ist.

Chirurgische Clips dieser Art sind beispielsweise aus der US 5,944,729 A (die als nächst liegender Stand der Technik angesehen wird) und der US 4,943,298 A bekannt.

Wesentlich bei den chirurgischen Clips der eingangs beschriebenen Art ist ihre Schließkraft, die präzise eingestellt sein muss und insbesondere bei der Verwendung des chirurgischen Clips als Implantat auch auf Dauer gegeben sein muss.

Die Herstellung der chirurgischen Clips der eingangs beschriebenen Art ist deswegen ein aufwändiger Vorgang.

Aufgabe der Erfindung ist es, einen chirurgischen Clip zu schaffen, der einfacher, insbesondere auch kostengünstiger herstellbar ist.

Diese Aufgabe wird erfindungsgemäß durch einen chirurgischen Clip mit den Merkmalen des Anspruchs 1 gelöst.

Die vorliegende Erfindung nutzt einen beim Spritzgießen in der Regel als unerwünscht beobachteten Effekt aus, um das federelastische Element des Clips im Spritzguss mit vorgegebener Schließkraft herzustellen, wobei über gezielt unterschiedliche Abkühlgeschwindigkeiten beim ersten und zweiten Wandbereich eine höhere Schwindung im ersten Wandbereich erzeugt wird als im zweiten Wandbereich.

Unterschiedliche Abkühlgeschwindigkeiten bei spritzgegossenen Kunststoffteilen rufen den Effekt des Verzugs des fertigen Bauteils hervor, wenn es aus der Spritzgussform entnommen wird. Die vorliegende Erfindung setzt gezielt diesen Effekt ein, um über die höhere Schwindung im ersten Wandbereich einen "Verzug" in dem federelastischen Element bzw. dessen Wandabschnitt nach der Entnahme aus der Spritzgussform zu erzeugen, auf dem aufbauend die von dem federelastischen Element ausgeübte Vorspannung und damit die vorgegebene Schließkraft des Clips sich einstellen lässt.

Bei dem erfindungsgemäßen Clip kann vorgesehen sein, dass die Clipbranchen aus einem metallischen Material gefertigt werden und das federelastische Element beispielsweise an die Clipbranchen angespritzt wird.

Die Clipbranchen können dann im Werkzeug bereits direkt aneinander anliegend positioniert werden, und ihren zweiten Enden wird beim Spritzgießen das federelastische Element bzw. dessen Wandabschnitt angespritzt.

Alternativ werden die Clipbranchen ebenfalls aus einem Kunststoffmaterial und dann vorzugsweise einteilig mit dem federelastischen Element im Spritzguss hergestellt. Im letzteren Fall können die beiden Clipbranchen mit ihren freien Enden selbstverständlich nicht aneinander anliegend im Spritzguss hergestellt werden, sondern sind über eine Wand der Spritzgussform voneinander getrennt und typischerweise parallel zueinander auf Abstand gehalten.

Trotzdem erlaubt die erfindungsgemäße Spritzgusstechnik, einen einteiligen Clip herzustellen, der im unbelasteten Zustand, nachdem er aus der Spritzgussform entnommen wurde, parallel aneinander anliegende Clipbranchen und eine vorgegebene Schließkraft aufweist.

Dieser erfindungsgemäße Clip hat dann zusätzlich den Vorteil, dass die beiden Clipbranchen beim Schließen des Clips mit ihren Spitzen zuerst in Kontakt miteinander kommen und der weitere Kontakt, bis die Clipbranchen insgesamt parallel aneinander anliegen, sich von der Spitze der Clipbranchen bis zu deren dem federelastischen Element benachbarten Enden hin entwickelt.

Dadurch lässt sich ein Herausschieben von Gewebe, das von dem Clip erfasst werden soll, verhindern.

Die Kunststoffe, die sich für die Herstellung des federelastischen Elements bzw. auch der Clipbranchen, soweit diese ebenfalls aus Kunststoff gefertigt sind, anbieten, sind im Hinblick auf die Verwendung als Implantat auszuwählen. In diesem Zusammenhang bieten sich insbesondere Hochtemperatur-Kunststoffe an wie Polyimide oder Polyketone, insbesondere Polyetherketone, weiter bevorzugt Polyetheretherketon (PEEK).

Bei der Auswahl der Kunststoffe können auch resorbierbare Kunststoffe berücksichtigt werden, wobei der Clip dann im Laufe der Zeit resorbiert wird und nicht als Fremdkörper im Körper des Patienten verbleibt.

Die unterschiedliche Schwindung des ersten Wandbereichs und des zweiten Wandbereichs des Wandabschnitts des federelastischen Elements kann insbesondere im Rahmen einer variothermen Spritzgießtechnik realisiert werden, wobei durch eine gezielte Abkühlung bzw. ein gezieltes Aufheizen oder eine Kombination von beidem die entsprechenden Temperaturprofile im Werkzeug bei der Fertigung des ersten und zweiten Wandbereichs des Wandabschnitts bereit gestellt werden können.

Bei einer weiter bevorzugten Ausführungsform der vorliegenden Erfindung ist vorgesehen, dass das federelastische Element an seiner den Clipbranchen abgewandten Seite, insbesondere anschließend an den zweiten Wandbereich, Anschlagelemente aufweist, welche beim Überführen des Clips aus der Ruhestellung in eine Applikationsstellung, bei der die freien Enden der Clipbranchen voneinander beabstandet sind, einen Anschlag bilden.

Damit wird verhindert, dass der chirurgische Clip bei der Applikation an einem Gefäß mit dem dafür typischerweise verwendeten Werkzeug in unzulässiger Weise verformt wird, was sich negativ für die Schließkraft, insbesondere die auf Dauer gegebene Schließkraft, des Clips auswirken würde.

Es muss deswegen bei der Handhabung der chirurgischen Clips darauf geachtet werden, dass diese nur in dem Bereich eine Verformung erfahren, in dem noch keine plastische Verformung des federelastischen Elements auftritt, so dass bei der Rückkehr in Richtung der Ruhelage der Clipbranchen die volle vorgegebene Schließkraft des Clips wirksam wird und bleibt.

Für eine Vielzahl chirurgischer Clips ist ein maximaler Öffnungswinkel der Clipbranchen in der Applikationsstellung von bis zu ca. 45 ° vorteilhaft.

Weiter bevorzugt können die Anschlagelemente komplementär ausgebildet sein, so dass sich damit eine gegenseitige Zentrierung der Anschlagelemente erzielen lässt, verbunden mit einer korrekten Ausrichtung der Clipbranchen zueinander in der geöffneten Applikationsstellung.

Dabei können die komplementär ausgebildeten Anschlagelemente bevorzugt so gestaltet werden, dass bei der Überführung der Clipbranchen in die Applikationsstellung eine laterale und/oder axiale Führung der Anschlagelemente resultiert.

Der Wandabschnitt des federelastischen Elements mit dem ersten Wandbereich, der mit einer höheren Schwindung gefertigt ist als der zweite Wandbereich, wird vorzugsweise eine im Wesentlichen hohlzylindrische Kontur aufweisen. Dieser Wandabschnitt kann auch Segment oder Teil eines ovalen Querschnitts sein.

Bei einer weiter bevorzugten Ausführungsform der Erfindung ist vorgesehen, dass die Anschlagelemente an ihren voneinander abgewandten Oberflächen Positionierelemente für ein Werkzeug aufweisen, so dass das Greifen des chirurgischen Clips mit einem applizierenden Werkzeug in einer vorgegebenen gegenseitigen Positionierung erfolgen kann.

Diese Positionierelemente können auch dazu dienen, eine sichere Verbindung zwischen dem Applikator und dem chirurgischen Clip herzustellen und so ein versehentliches Verlieren des Clips aus dem Werkzeug während der Applikation zu verhindern.

Diese Positionierelemente sind insbesondere als Vertiefungen, insbesondere auch als Bohrungen, in den Anschlagelementen vorgesehen.

Der chirurgische Clip wird insbesondere als Aneurysmen-Clip ausgebildet, insbesondere auch als cerebraler Aneurysmen-Clip.

Die Erfindung betrifft des Weiteren ein Verfahren zur Herstellung eines chirurgischen Clips wie oben dargestellt, wobei ein Spritzgusswerkzeug zum Einsatz kommt, welches unterschiedliche Temperaturführungen in der Wandung des Spritzgusswerkzeuges benachbart zum ersten und zweiten Wandbereich erlaubt, so dass der erste Wandbereich des federelastischen Elements des Clips mit einer höheren Schwindung gefertigt werden kann.

Diese und weitere Vorteile der Erfindung werden im Weiteren anhand der Zeichnungen noch näher erläutert. Es zeigen im Einzelnen:
- Figur 1A und B:: einen erfindungsgemäßen chirurgischen Clip in Ruhe- bzw. Applikationsstellung;
- Figur 2:: den chirurgischen Clip der Figur 1A in unterschiedlichen Öffnungswinkeln; und
- Figur 3:: eine schematische Darstellung eines Wandabschnitts des chirurgischen Clips.

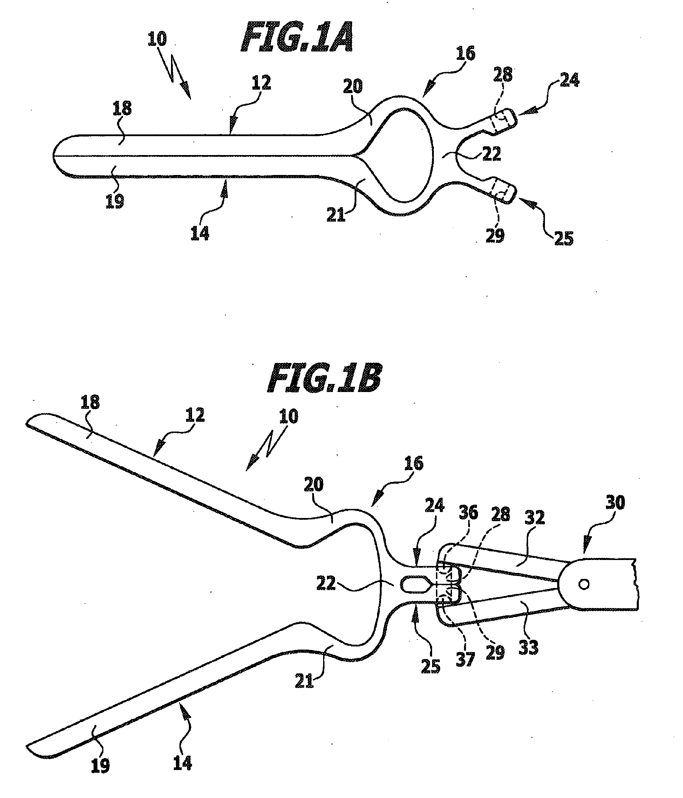

Figur 1A zeigt einen erfindungsgemäßen chirurgischen Clip 10 mit einer ersten Clipbranche 12, einer zweiten, dazu parallel angeordneten Clipbranche 14 und einem federelastischen Element 16, welches die beiden Clipbranchen miteinander verbindet.

Die Clipbranchen 12 und 14 weisen freie Enden 18, 19 auf, die parallel aneinander anliegen, während die den ersten freien Enden 18, 19 entgegengesetzt liegenden zweiten Enden 20, 21 einstückig mit dem Federelement 16 bzw. dessen Wandabschnitt 22 verbunden sind.

Das Federelement 16 weist auf seiner den Clipbranchen 12, 14 abgewandten Seite Anschlagelemente 24, 25 auf, die senkrecht von dem Wandabschnitt 22 abstehen und zwischen einander einen Winkel von ungefähr 45 ° bilden.

Die Anschlagelemente 24, 25 weisen in ihrem der Wandung 22 abgelegenen freien Ende Bohrungen 28, 29 auf, die als Positionierhilfen für ein Applikatorwerkzeug dienen, wie dies im Folgenden anhand der Figur 1B noch gezeigt und beschrieben wird.

Die Figur 1B zeigt den Clip 10 in einer Applikationsstellung, bei der der Clip 10 an seinen Anschlagelementen 24, 25 von einem Clipapplikator 30 mit seinen Greifwerkzeugen 32, 33 erfasst wird.

Die Greifwerkzeuge 32, 33 zwingen die beiden Anschlagelemente 24, 25 in direkten Kontakt miteinander, worauf sich die Clipbranchen 12, 14 in einem Winkel von ca. 45 ° öffnen (Applikationsstellung). Aufgrund der Anschlagelemente 24, 25 ist sichergestellt, dass der Clip 10, insbesondere der Wandabschnitt 22 des federelastischen Elements 16, nicht über den zulässigen Bereich hinaus verformt wird, so dass die Verformung sicher im elastischen Bereich vorgenommen wird und keine plastische Verformung des federelastischen Elements 16 vorkommen kann.

Um das Greifen des Clips 10 zu erleichtern und den Kontakt zwischen dem Applikatorwerkzeug 30 und dem Clip 10 sicherer zu machen, sind an den Greifwerkzeugen 32, 33 zylindrische Vorsprünge 36, 37 vorgesehen, die in die Bohrungen 28, 29 der Anschlagelemente 24, 25 eingreifen können und so eine sichere Verbindung zwischen Clip 10 und Applikator 30 herstellen und sichern.

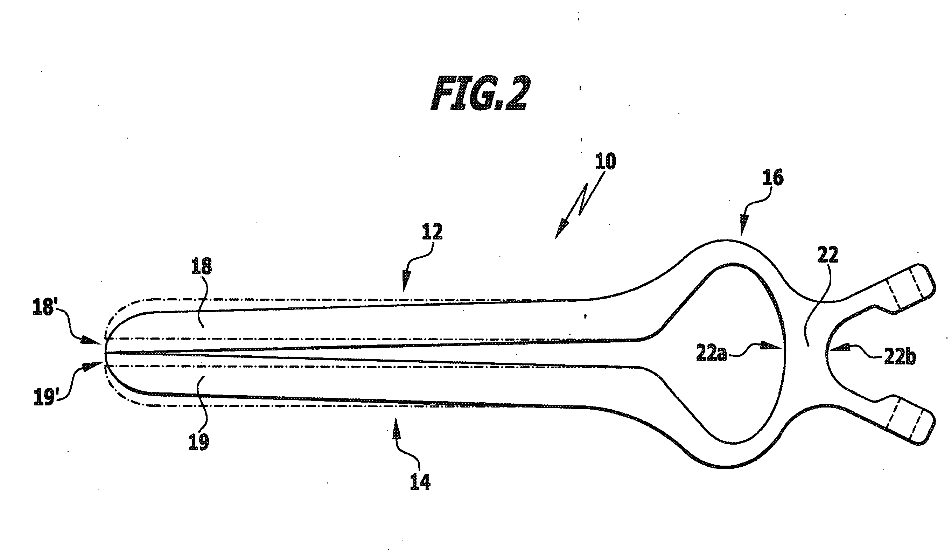

Figur 2 zeigt den erfindungsgemäßen Clip 10 schematisiert in zwei unterschiedlichen Stellungen, wobei die strichpunktierte Darstellung der Clipbranchen 12, 14 deren gegenseitige Position im Spritzgusswerkzeug andeutet.

Wird der erfindungsgemäße Clip 10 dem Spritzgusswerkzeug entnommen, nähern sich zunächst die freien Enden 18, 19 der beiden Clipbranchen 12, 14 einander an, wobei ein erster Kontakt der Clipbranchen 12, 14 an deren äußerstem, dem federelastischen Element 16 abgewandten Ende, den Spitzen 18', 19' zustande kommt. Im weiteren Verlauf wird aufgrund des in dem Wandabschnitt 22 vorhandenen unterschiedlichen Schwindungseffekts auf Seiten eines ersten Wandbereichs 22a gegenüber einem zweiten Wandbereich 22b ein vollständiges Schließen der freien Enden 18, 19 der Clipbranchen 12, 14 erzielt, so dass diese vollflächig aneinander anliegen (Ruhestellung), wie in Figur 1A gezeigt.

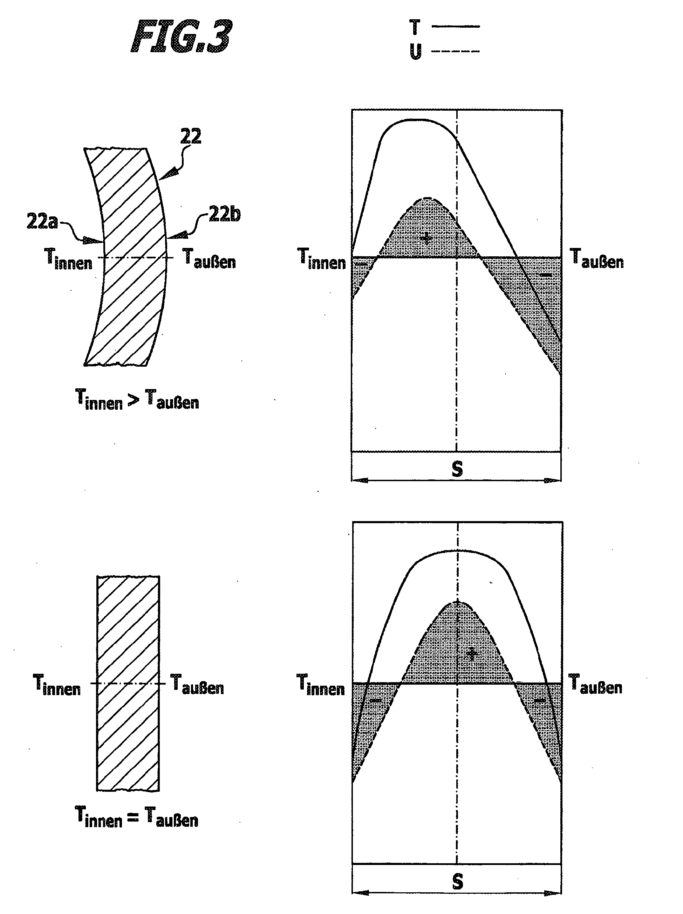

Der Effekt der Erzielung unterschiedlicher Schwindung in dem ersten und dem zweiten Wandbereich des Wandabschnitts 22 ist in Figur 3 schematisch veranschaulicht.

Erfindungsgemäß wird die Temperatur beim Abkühlen der Spritzgussmasse im Werkzeug im Bereich des Wandabschnitts 22 des federelastischen Elements 16 so vorgenommen, dass die Temperatur des zweiten Wandbereichs 22b niedriger ist als die Temperatur auf Seiten des ersten Wandbereichs 22a, so dass sich eine höhere Schwindung auf Seiten des Wandbereichs 22a einstellt. Dadurch ergibt sich dann bei der Entformung des erfindungsgemäßen chirurgischen Clips 10 aus der Spritzgussform eine Vorspannung entsprechend einer vorgegebenen Schließkraft.

Über die Wahl der Innen- und Außentemperatur bzw. der unterschiedlichen Schwindung in dem ersten und dem zweiten Wandbereich kann die Vorspannung des federelastischen Elements und damit die Schließkraft des Clips 10 eingestellt werden.

Die genaue Wahl der Temperaturen ist zum Einen abhängig von der Dicke S des Wandbereichs 22, ebenso wie von der gewünschten Schließkraft und schließlich auch von dem verarbeiteten Kunststoffmaterial.

Nur zum Vergleich ist in der Figur 3 unten dargestellt, wie sich bei ansonsten identischen Parametern ein Spritzgussteil verhält, wenn die Temperaturen T innen und außen beim Abkühlen der Kunststoffmasse in der Spritzgussform gleich gehalten werden.

Es entsteht keine unterschiedliche Schwindung im inneren und im äußeren Teil und somit gleichen sich die auftretenden Spannungen U in dem Wandabschnitt auf, und der Wandabschnitt selbst zeigt keinen Verzug bzw. kann keine Vorspannung erzeugen.

## Patentansprüche

1. Chirurgischer Clip (10), insbesondere zur Verwendung als Implantat, mit zwei Clipbranchen (12; 14) und einem federelastischen Element (16), über das die beiden Clipbranchen (12; 14) schwenkbar miteinander verbunden sind, wobei die beiden Clipbranchen (12; 14) jeweils ein erstes freies Ende (18; 19) aufweisen, wobei die Clipbranchen (12; 14) an ihrem dem freien Ende (18; 19) entgegen gesetzten zweiten Ende (20; 21) an einem Wandabschnitt (22) des federelastischen Elements (16) angeschlossen gehalten sind, der im Wesentlichen quer zur Längsrichtung der Clipbranchen (12; 14) angeordnet ist, der sich zwischen den zweiten Enden (20; 21) der Clipbranchen (12; 14) erstreckt und der einen ersten den Clipbranchen (12; 14) zugewandten Wandbereich (22a) und einen zweiten den Clipbranchen abgewandten Wandbereich (22b) aufweist, **dadurch gekennzeichnet, dass** das federelastische Element (16) im Spritzguss aus einem Kunststoffmaterial gefertigt ist, wobei der erste Wandbereich (22a) des Wandabschnitts (22) mit einer höheren Schwindung gefertigt ist als der zweite Wandbereich (22b), wodurch die freien Enden (18; 19) der beiden Clipbranchen (12; 14) in einer Ruhestellung des Clips (10) mit einer vorgegebenen Schließkraft durch das federelastische Element (16) parallel aneinander anliegend gehalten sind.

2. Chirurgischer Clip (10) nach Anspruch 1, **dadurch gekennzeichnet, dass** die Clipbranchen (12; 14) aus einem metallischen Material gefertigt sind und das federelastische Element (16) an die Clipbranchen (12; 14) angespritzt ist.

3. Chirurgischer Clip (10) nach Anspruch 1, **dadurch gekennzeichnet, dass** die Clipbranchen (12; 14) aus einem Kunststoffmaterial gefertigt und vorzugsweise einteilig mit dem federelastischen Element (16) im Spritzguss hergestellt sind.

4. Chirurgischer Clip (10) nach einem der Ansprüche 1 bis 3, **dadurch gekennzeichnet, dass** das federelastische Element (16) an seiner den Clipbranchen (12; 14) abgewandten Seite Anschlagelemente (24; 25) aufweist, welche beim Überführen des Clips (10) aus der Ruhestellung in eine Applikationsstellung, bei der die freien Enden (18; 19) der Clipbranchen (12; 14) voneinander beabstandet sind, einen Anschlag bilden.

5. Chirurgischer Clip (10) nach Anspruch 4, **dadurch gekennzeichnet, dass** der Öffnungswinkel der Clipbranchen (12; 14) in der Applikationsstellung auf einen Wert von ca. 45 ° begrenzt ist.

6. Chirurgischer Clip (10) nach Anspruch 4 oder 5, **dadurch gekennzeichnet, dass** die Anschlagelemente (24; 25) komplementär ausgebildet sind.

7. Chirurgischer Clip (10) nach Anspruch 6, **dadurch gekennzeichnet, dass** die komplementär ausgebildeten Anschlagelemente (24; 25) so ausgebildet sind, dass bei der Überführung der Clipbranchen (12; 14) in die Applikationsstellung eine laterale und/oder axiale Führung der Anschlagelemente (24; 25) resultiert.

8. Chirurgischer Clip (10) nach einem der Ansprüche 1 bis 7, **dadurch gekennzeichnet, dass** die Clipbranchen (12; 14) bei der Überführung aus der Applikationsstellung in die Ruhelage zuerst mit ihren freien Enden (18; 19) miteinander in Kontakt kommen.

9. Chirurgischer Clip (10) nach einem der Ansprüche 1 bis 8, **dadurch gekennzeichnet, dass** der Wandabschnitt (22) des federelastischen Elements (16) eine im Wesentlichen hohlzylindrische Kontur aufweist.

10. Chirurgischer Clip (10) nach einem der Ansprüche 1 bis 9, **dadurch gekennzeichnet, dass** die Anschlagelemente (24; 25) an ihren außen liegenden Oberflächen Positionierelemente (28; 29) für ein Werkzeug aufweisen.

11. Chirurgischer Clip (10) nach einem der Ansprüche 1 bis 10, **dadurch gekennzeichnet, dass** der Clip (10) als Aneurysmen-Clip, insbesondere als cerebraler Aneurysmen-Clip ausgebildet ist.

12. Chirurgischer Clip (10) nach einem der Ansprüche 1 bis 11, **dadurch gekennzeichnet, dass** das Kunststoffmaterial des federelastischen Elements (16) und gegebenenfalls der Clipbranchen (12; 14) ein Polyimid oder ein Polyetherketon, insbesondere ein PEEK-Material ist.

13. Chirurgischer Clip (10) nach einem der Ansprüche 1 bis 11, **dadurch gekennzeichnet, dass** das Kunststoffmaterial des federelastischen Elements (16) und gegebenenfalls der Clipbranchen (12; 14) ein resorbierbares Kunststoffmaterial ist.

14. Verfahren zur Herstellung eines chirurgischen Clips (10) gemäß einem der Ansprüche 1 bis 13, **dadurch gekennzeichnet, dass** bei der Fertigung des federelastischen Elements (16) des Clips (10) ein schmelzflüssiges Kunststoffmaterial in eine Form gegeben und abgekühlt wird, wobei die Form in ihrem den Wandabschnitt (22) des federelastischen Elements (16) ausbildenden Bereich auf Seiten des ersten Wandbereichs (22a) des Wandabschnitts (22) beim Abkühlen des Kunststoffmaterials auf einer höheren Temperatur gehalten wird als auf Seiten des zweiten Wandbereichs (22b).

## Claims

1. Surgical clip (10), in particular for use as an implant, comprising two clip arms (12; 14) and a resiliently flexible element (16) via which the two clip arms (12; 14) are pivotally connected to each other, wherein the two clip arms (12;14) each have a first free end (18; 19), wherein the clip arms (12; 14) at a second end (20; 21) thereof opposite the free end (18; 19) are held connected to a wall section (22) of the resiliently flexible element (16) which is arranged substantially transverse to the longitudinal direction of the clip arms (12; 14), extends between the second ends (20; 21) of the clip arms (12; 14) and has a first wall portion (22a) facing towards the clip arms (12; 14) and a second wall portion (22b) facing away from the clip arms (12; 14), **characterized in that** the resiliently flexible element (16) is made of a plastics material by injection moulding, wherein the first wall portion (22a) of the wall section (22) is produced with a greater shrinkage than the second wall portion (22b), whereby the first free ends (18;19) of the two clip arms (12; 14) are held in parallel to and in contact with each other with a predetermined closing force by the resiliently flexible element (16) when the clip (10) is in a rest position.

2. Surgical clip (10) in accordance with claim 1, wherein the clip arms (12; 14) are made of a metallic material and the resiliently flexible element (16) is integrally formed on the clip arms (12; 14) by injection moulding.

3. Surgical clip (10) in accordance with claim 1, wherein the clip arms (12; 14) are made of a plastics material and are preferably formed by injection-moulding them in one piece with the resiliently flexible element (16).

4. Surgical clip (10) in accordance with any one of claims 1 to 3, wherein the resiliently flexible element (16) has on the side thereof facing away from the clip arms (12; 14) stop elements (24; 25) which form a stop when the clip (10) is transferred from the rest position to an application position in which the free ends (18; 19) of the clip arms (12; 14) are spaced apart from each other.

5. Surgical clip (10) in accordance with claim 4, wherein the opening angle of the clip arms (12; 14) in the application position is limited to a value of approximately 45°.

6. Surgical clip (10) in accordance with claim 4 or 5, wherein the stop elements (24; 25) are configured to be of complementary form.

7. Surgical clip (10) in accordance with claim 6, wherein the complementary-configured stop elements (24; 25) are configured such that at least one of a lateral and an axial guiding of the stop elements (24; 25) results when the clip arms (12; 14) are transferred to the application position.

8. Surgical clip (10) in accordance with any one of claims 1 to 7, wherein the clip arms (12; 14) come into contact with each other with their free ends (18; 19) first when transferred from the application position to the rest position.

9. Surgical clip (10) in accordance with any one of claims 1 to 8, wherein the wall section (22) of the resiliently flexible element (16) has a substantially hollow-cylindrical contour.

10. Surgical clip (10) in accordance with any one of claims 1 to 9, wherein the stop elements (24, 25) have on the exterior surfaces thereof positioning elements (28; 29) for a tool.

11. Surgical clip (10) in accordance with any one of claims 1 to 10, wherein the clip (10) is configured as an aneurysm clip, in particular as a cerebral aneurysm clip.

12. Surgical clip (10) in accordance with any one of claims 1 to 11, wherein the plastics material of the resiliently flexible element (16) and optionally of the clip arms (12; 14) is a polyimide or a polyetherketone, in particular a PEEK material.

13. Surgical clip (10) in accordance with any one of claims 1 to 11, wherein the plastics material of the resiliently flexible element (16) and optionally of the clip arms (12; 14) is a resorbable plastics material.

14. Method for producing a surgical clip (10) in accordance with any one of claims 1 to 13, wherein in the process of manufacturing the resiliently flexible element (16) of the clip (10), a molten liquid plastics material is fed into a mould and cooled, wherein as the plastics material cools, the mould is held in the region thereof forming the wall section (22) of the resiliently flexible element (16) at a higher temperature on the side of the first wall portion (22a) than on the side of the second wall portion (22b).

## Revendications

1. Clamp ou clip chirurgical (10), notamment destiné à être utilisé en tant qu'implant, et comprenant deux branches de clamp (12; 14) et un élément à élasticité de ressort (16) par l'intermédiaire duquel les deux branches de clamp (12; 14) sont reliées l'une à l'autre de manière articulée,
dans lequel les deux branches de clamp (12; 14) présentent chacune une première extrémité libre (18; 19), ces extrémités libres étant, dans une position de repos du clamp (10), maintenues appliquées parallèlement l'une contre l'autre avec une force de fermeture prédéterminée, sous l'effet de l'élément à élasticité de ressort (16), et
dans lequel les branches de clamp (12; 14) sont, à leur deuxième extrémité (20; 21) opposée à l'extrémité libre (18; 19), maintenues raccordées à un tronçon de paroi (22) de l'élément à élasticité de ressort (16), qui est agencé sensiblement transversalement à la direction longitudinale des branches de clamp (12; 14), s'étend entre les deuxièmes extrémités (20; 21) des branches de clamp (12; 14), et présente une première zone de paroi (22a) dirigée vers les branches de clamp (12; 14) et une deuxième zone de paroi (22b) située du côté opposé à celui dirigé vers les branches de clamp,
**caractérisé en ce que** l'élément à élasticité de ressort (16) est fabriqué par moulage par injection en une matière plastique, la première zone de paroi (22a) du tronçon de paroi (22) étant fabriquée avec une contraction de retrait plus élevée que la deuxième zone de paroi (22b).

2. Clamp chirurgical (10) selon la revendication 1, **caractérisé en ce que** les branches de clamp (12; 14) sont fabriquées en un matériau métallique, et l'élément à élasticité de ressort (16) est moulé par injection sur les branches de clamp (12; 14).

3. Clamp chirurgical (10) selon la revendication 1, **caractérisé en ce que** les branches de clamp (12; 14) sont fabriquées en une matière plastique, et sont de préférence fabriquées d'un seul tenant avec l'élément à élasticité de ressort (16) par un procédé de moulage par injection.

4. Clamp chirurgical (10) selon l'une des revendications 1 à 3, **caractérisé en ce que** l'élément à élasticité de ressort (16) présente, sur son côté opposé à celui où se trouvent les branches de clamp (12; 14), des éléments de butée (24; 25), qui, lors du transfert du clamp (10) de la position de repos à une position d'application dans laquelle les extrémités libres (18; 19) des branches de clamp (12; 14) sont écartées l'une de l'autre, forment une butée.

5. Clamp chirurgical (10) selon la revendication 4, **caractérisé en ce que** l'angle d'ouverture des branches de clamp (12; 14) est limité à une valeur d'environ 45° dans la position d'application.

6. Clamp chirurgical (10) selon la revendication 4 ou la revendication 5, **caractérisé en ce que** les éléments de butée (24; 25) sont d'une configuration complémentaire.

7. Clamp chirurgical (10) selon la revendication 6, **caractérisé en ce que** les éléments de butée (24; 25) de configuration complémentaire sont réalisés de manière à ce que, lors du transfert des branches de clamp (12; 14) dans la position d'application, il en résulte un guidage latéral et/ou axial des éléments de butée (24; 25).

8. Clamp chirurgical (10) selon l'une des revendications 1 à 7, **caractérisé en ce que** les branches de clamp (12; 14), lors du transfert de la position d'application à la position de repos, entrent tout d'abord en contact réciproque, avec leurs extrémités libres (18; 19).

9. Clamp chirurgical (10) selon l'une des revendications 1 à 8, **caractérisé en ce que** le tronçon de paroi (22) de l'élément à élasticité de ressort (16) présente un contour sensiblement cylindrique creux.

10. Clamp chirurgical (10) selon l'une des revendications 1 à 9, **caractérisé en ce que** les éléments de butée (24; 25) présentent, sur leurs surfaces situées à l'extérieur, des éléments de positionnement (28; 29) pour un outil.

11. Clamp chirurgical (10) selon l'une des revendications 1 à 10, **caractérisé en ce que** le clamp (10) est réalisé en tant que clamp d'anévrisme, notamment en tant que clamp d'anévrisme cérébral.

12. Clamp chirurgical (10) selon l'une des revendications 1 à 11, **caractérisé en ce que** la matière plastique de l'élément à élasticité de ressort (16) et, le cas échéant, des branches de clamp (12; 14), est un matériau du type polyimide ou un polyéthercétone, notamment un PEEK.

13. Clamp chirurgical (10) selon l'une des revendications 1 à 11, **caractérisé en ce que** la matière plastique de l'élément à élasticité de ressort (16) et, le cas échéant, des branches de clamp (12; 14), est une matière plastique résorbable.

14. Procédé de fabrication d'un clamp chirurgical (10) selon l'une des revendications 1 à 13, **caractérisé en ce que** lors de la fabrication de l'élément à élasticité de ressort (16) du clamp (10), une matière plastique liquide en fusion est amenée à un moule et est refroidie, le moule, dans sa région formant le tronçon de paroi (22) de l'élément à élasticité de ressort (16), étant maintenu lors du refroidissement de la matière plastique, au niveau du côté de la première zone de paroi (22a) du tronçon de paroi (22), à une température plus élevée que sur le côté de la deuxième zone de paroi (22b).
